# EUROPEAN PATENT APPLICATION

(11) **EP 1 932 532 A1**
(43) Date of publication of application: **18.06.2008**
(21) Application number: 06783091.9
(22) Date of filing: 28.08.2006
(51) Int. Cl.: A61K 36/75, A61K 9/70, A61K 36/18, A61K 36/28, A61K 36/53, A61K 36/73, A61K 47/04, A61K 47/06, A61K 47/14, A61K 47/34, A61K 47/36, A61K 47/44, A61P 3/04

(54) **DIETING COMPOSITION COMPRISING PLANT-DERIVED ESSENTIAL OIL AS ACTIVE INGREDIENT, SHEET-TYPE DIETING COMPOSITION COMPRISING THE COMPOSITION, TRANSDERMAL PHARMACEUTICAL PREPARATION FOR DIETING, AND METHODS FOR PRODUCTION OF THE PREPARATION**

(30) Priority: 31.08.2005 JP 2005250735
(71) Applicant: Nature Technology, Inc., Iwamizawa-shi Hokkaido 068-0853, (JP)
(72) Inventor: KARITA, Takeshi, Hokkaido 069-0824 (JP)
(74) Representative: Gille Hrabal Struck Neidlein Prop Roos
(86) International application number: PCT/JP2006/316862
(87) International publication number: WO 2007/026645

(57) **Abstract**

The present invention provides a composition for diet comprising an essential oil adsorbent is coated with neroli oil to prepare an essential oil-coated particle, and then the particle is covered with a essential oil adsorbing and desorbing agent to prepare the carbon-coated particle (NR), the carbon-coated particle similarly prepared by using jasmin oil instead of neroli oil (JS), the carbon-coated particle similarly prepared by using rose oil instead of neroli oil (RO), and/or the carbon-coated particle similarly prepared by using cineol type Eucalyptus oil instead of neroli oil (CE), and the agent for diet comprising thereof as active ingredients. Accordingly, the agent for treating adipositas having high safety, of which composition ratio can be changed depending on a symptom of adipositas, is provided.

## Description

### FIELD OF THE INVENTION

The present invention relates to a composition having diet effect which comprising essential oils derived from plants as active ingredients, a sheet type composition for the diet comprising thereof, a percutaneous pharmaceutical agent for the diet comprising thereof, and a method for producing thereof.

### BACKGROUND ART

Recently, life style and custom including for taking westernized meals becomes popular in Japan, and it promotes the number of patients of cardiovascular disease such as myocardial infarction, and cerebral infarction. The cardiovascular disease holds the foremost place leading case of death in Japan. The increment of the number of cardiovascular patients almost synchronizes that of arteriosclerosis patients, however a variety of risk factor relates to the increment of the cardiovascular patients.

Recent years, it is clarified that multiple risk factor syndrome, which is defined that a disease on which many risk factors concentrates to one patient, is a key for appearing the arteriosclerosis.

Several years ago, National Cholesterol Education Program (NCEP) gave a name "metabolic syndrome" to the disease and published its criterion. In so-called metabolic syndrome, obesity, diabetes, hyperlipidemia, and hypertension are complicating, it is considered that these become main causes for the cardiovascular disease as described above.

On the other hand, in advanced countries, numbers of obese individual depending on nutrient excess are exponentially increasing. This is caused by reduced consumption energy, depending on the increase of calories intake from abundance of food, and the reduction of people's burden of housework and other works based on the introduction of electrical appliance.

Among the term obesity in general, the obesity which requires to loose weight and dietary restriction is referred to as adipositas, and its criterion is decided by Japanese Society for the Study of Obesity. Particularly, the obesity that accumulattes fat around viscus is accompanied by diabetes, hyperlipidemia and hypertension, and this clinical condition is complied with that of metabolic syndrome.

Fat people are not necessarily ephemeral. However, as the increase of degree of obesity, it is statistically demonstrated that the mortality rate of such people is higher compared with normal body weight; about 1.20 for the people having the degree of obesity ≥ 20 %, about 1.50 for the people having the degree of obesity ≥ 40 %, about 2.0 for the people having the degree of obesity ≥ 50 %.

Such increases of the mortality rate is derived from that the fat people complicate various disease as described above. Therefore, the medical treatment for the obesity leads directly to that for the complicating diseases and improve their conditions of health.

As the criterion for the obesity, BMI (body mass index) is internationally used in adults. BMI is used for evaluating the degree of obesity by using height and weight to be added, subtracted, multiplied and divided. Based on the evaluation that BMI reflects amounts of body fat, WHO proposed a classification of the obesity, and contracting states of the International Association for the Study of Obesity decided that they used the classification, and the Japanese Society for the Study of Obesity also decided to employ BMI.
The degree of obesity is classified by using BMI as the following table 1.

**(Table 1)**

| BMI | Evaluation | WHO standard |
|---|---|---|
| <18.5 | Underweight | Underweight |
| 18.5 ≤ ~ <25 | Normal weight | Normal weight |
| 25 ≤ ~ <30 | Obese (Class 1) | Preobese |
| 30 ≤ ~ <35 | Obese (Class 2) | Obese class I |
| 35 ≤ ~ <40 | Obese (Class 3) | Obese class II |
| 40 ≤ | Obese (Class 4) | Obese class III |

Adult diseases such as diabetes, hypertension, hyperlipidemia, gout, and arteriosclerosis, being associated strongly with the obesity, are shown in the table 2.

**(Table 2)**

| | |
|---|---|
| | |
| 1 | Diabetes, abnormal glucose tolerance |
| 2 | Abnormal lipid metabolism |
| 3 | Hypertension |
| 4 | Hyperuricemia, gout |
| 5 | Coronary artery disease, myocardial infarction, angina pectoris |
| 6 | Sleep apenea syndrome, Pickwick syndrome |
| 7 | Fatty liver |
| 8 | Cerebral infarction: cerebral thromboemblism, transient ischemia |
| 9 | Orthopedic [orthopaedic] surgery disease: degenerative arthritis(osteoarthritis), lumbar disease |
| 10 | Abnormality of menstruation, inability to conceive(infertility) |

Adult disease as described above is not necessarily associated with the obesity, but its risk depends on the stored fat distribution.

The obesity is classified into upper-body obesity putting fat on the abdomen, and lower-body obesity putting fat mainly on the bottom; the upper-body obesity is sometimes called as man-pattern obesity, abdominal obesity or apple pattern obesity, and the lower-body obesity is sometimes called as female pattern obesity or pear-shaped obesity. Between them, the risk being associated with the adult disease is higher in the upper-body obesity.
The upper-body obesity or the lower-body obesity is decided by using waistline size, because the waistline size as high correlation between the associated diseases with obesity; a man whose waistline size is not less than 85 cm, a woman's waistline size is not less than 90 cm are decided that they are the upper-body obesity.

Alternatively, the upper body obesity is classified into visceral fat accumulation type obesity (the visceral fat obesity), and subcutaneous fat accumulation type obesity in which the fat is accumulated under the abdominal wall (the subcutaneous fat obesity), depending on the site of the fat accumulated on the upper-body. When the area of the visceral fat is not less than 100 cm², it is decided that the person is the visceral fat obesity.

Based on the standard as described above, when either of the followings are fulfilled: 1) the case that BMI is not less than 25 and being already associated with the disease described on table 2, or 2) the case suspected having the upper-body obesity and being confirmed that the visceral fat obesity by using CT scan, the case is defined as the "adipositas" which need treatments from the medical standpoint. Except the above-mentioned case, they are defined as general "obesity".

A pharmaceutical agent for treatment the adipositas authorized by Health, Labour and Welfare Ministry is only Mazindol (Sanorex, Registered trademark, Novartis Pharma K.K.).

### Disclosure of the Invention

### Problems to be solved by the invention

The use of Mazindol is limited to patients having severe obesity whose BMI is not less that 35 (the degree of obesity is not less than 70 %). As a result, at present in Japan, there is no obesity remedy for fat people whose obesity is mild to moderate.
Alternatively, it has gradual weight loss of average about 4.5 kg in three month is reported (see non-patent document 1. It is referred to as Prior art 1, herein below).

As side effects derived from Mazindol, there are not serious ones except cotton mouth, astriction, indigestible feeling. Therefore, it has highly safe from the side effect point of view. On the other hand, since the effect of Mazindol is not persistent, its use more than 3 month is prohibited, and it is said that there are many cases gained their weight after coming off Mazindol.
Accordingly, there is a strong interest for the obesity remedy for fat people whose obesity is mild to moderate with highly safety.

[Non-patent document 1] Adipositas Text, p. 147, published on September 1, 2004, 2nd edition, Nankodo Co., Ltd.

### Means for Solving the Problem

Under these situations, the inventors of the present invention found that combinations of certain essential oils have diet effect when the combination is used in percutaneous type pharmaceutical agent, and then they completed the present invention.
That is, the present invention is a composition for diet comprising a carbon-coated particle which is produced by covering an essential oil adsorbent with neroli oil to prepare an essential oil-coated particle, and then further covering the particle by an essential oil adsorbing and desorbing regulator to prepare a carbon-coated particle (NR); and a carbon-coated particle which is produced by covering an essential oil adsorbent with jasmine oil to prepare an essential oil-coated particle, and then further covering the particle by an essential oil adsorbing and desorbing regulator to prepare a carbon-coated particle (JS), and a percutaneous type pharmaceutical agent for diet comprising thereof as active ingredients.
Since the percutaneous type pharmaceutical agent for diet comprises the essential oils from plants as the active ingredients, it neither generates any side-effects derived from the conventional pharmaceutical agent for the diet described above nor addiction.

Here, the composition preferably further comprises a carbon-coated particle wherein a hydrophilic resin as the essential oil adsorbent is coated by lavender oil to prepare the neroli oil-coated particle, and then it is covered by the carbon fine powder as the essential oil adsorbing and desorbing regulator to prepare a carbon-coated particle (LV), except the carbon-coated particle wherein a hydrophilic resin as the essential oil adsorbent is coated by neroli oil to prepare the neroli oil-coated particle, and then it is covered by the carbon fine powder as the essential oil adsorbing and desorbing regulator to prepare a carbon-coated particle (NR), and a carbon-coated particle which is produced by covering an essential oil adsorbent with jasmine oil to prepare an essential oil-coated particle, and then further covering the particle by an essential oil adsorbing and desorbing regulator to prepare a carbon-coated particle (JS).
By preparing a composition combined these carbon-coated particles, the composition shows far higher diet effect, comparing the case that the carbon-coated particles are solely used in the composition.

Furthermore, addition to the carbon-coated particles (LV), the composition of the present invention preferably further comprises the carbon-coated particle which is produced by covering an essential oil adsorbent with rose oil to prepare an essential oil-coated particle, and then further covering the particle by an essential oil adsorbing and desorbing regulator to prepare a carbon-coated particle (RO), and the carbon-coated particle which is produced by covering an essential oil adsorbent with lemon oil to prepare an essential oil-coated particle, and then further covering the particle by an essential oil adsorbing and desorbing regulator to prepare a carbon-coated particle.
By preparing a composition combined these carbon-coated particles, the composition shows far higher diet effect, comparing the case that the carbon-coated particles are solely used in the composition.

Furthermore, addition to the carbon-coated particle (NR), the carbon-coated particle (JS), the carbon-coated particle (RO), and the carbon-coated particle (LM), the composition of the present invention preferably further comprises a carbon-coated particle coated an essential oil adsorbent with any one of essential oil selected from the group consisting of cineol type Eucalyptus oil, gai oil, and sage oil, to prepare an essential oil-coated particle, and then further covering the particle by an essential oil adsorbing and desorbing regulator to prepare a carbon-coated particle (CE).
By preparing a composition combined these carbon-coated particles, the composition shows higher diet effect, comparing the case that the carbon-coated particle (LV) is solely used in the composition, or the combination of two of the carbon-coated particle (LV) and (RO) are used in the composition.

Alternatively, in the composition for diet of the present invention, an amount of neroli oil used to prepare said carbon-coated particle is in the range from 9 to 21 weight % against a total amount of essential oils used to prepare the composition; and the amount of jasmine oil used to prepare said carbon-coated particle is in the range from 9 to 15 weight % against a total amount of essential oils used to prepare the composition.

Here, it is preferable that the amount of lavender oil used to prepare said carbon-coated particle (LV) is in the range from 65 to 75 weight % against a total amount of essential oils used to prepare the composition; the amount of neroli oil used to prepare said carbon-coated particle (NR) is in the range from 15.5 to 20.5 weight % against a total amount of essential oils used to prepare the composition; and the amount of jasmine oil used to prepare said carbon-coated particle (JS) is in the range from 9.5 to 14.5 weight % against a total amount of essential oils used to prepare the composition.

In the composition for diet, it is preferable that the amount of lavender oil used to prepare said carbon-coated particle or that of any one of essential oil selected from the group consisting of cineol type Eucalyptus oil, gai oil, and sage oil, is in the range from 32 to 42 weight % against a total amount of essential oils used to prepare the composition; the amount of neroli oil used to prepare said carbon-coated particle is in the range from 10 to 12 weight % against a total amount of essential oils used to prepare the composition; the amount of jasmine oil used to prepare said carbon-coated particle is in the range from 10 to 12 weight % against a total amount of essential oils used to prepare the composition; the amount of lemon oil used to prepare said carbon-coated particle is in the range from 24.5 to 27 weight % against a total amount of essential oils used to prepare the composition; and the amount of rose oil used to prepare said carbon-coated particle is in the range from 13.5 to 17 weight % against a total amount of essential oils used to prepare the composition.

In a sheet type composition for diet of the present invention, (a) a combination of the carbon-coated particles as described above, (b) the essential oil adsorbing and desorbing agent, (c) the released water absorption agent, (d) a base material for sheet forming which includes an exothermic agent, a heat transfer inhibitor, and an absorption promoter. By using such constitution for the percutaneous type agent, it enables to release the essential oil from the plants as the active ingredients in the sustained manner.

Also, by employing such a constitution, the composition for the sheet type percutaneous pharmaceutical agent for diet, wherein the active ingredients are homogenously distributed, can be obtained. By cutting the composition in the desirable size, the composition including the suitable amounts of the active ingredients can be conveniently produced.

Here, the preferable essential oil adsorbent is a polyvinyl alcohol type water absorption resin of which saponification value is from 98.0 to 98.5. The preferable released water absorption agent is an acrylic type water-absorptive resin, and it is preferable that the resin is capable of absorbing 400 to 800 times its volume of water based on the volume of the water-absorptive acrylic resin.

Furthermore, the an essential oil adsorbing and desorbing regulator is a porous carbon material having surface area from 200 to 800 m²/g; an exothermic agent composed of an artificial zeolite having a pore size from 0.1 to 0.8 nm. The heat transfer inhibitor is preferably composed of a polysaccharide compound.

Further, the absorption promoter is preferably monoterpene compounds, and the monoterpene compounds is preferably L-menthol or limonene. In addition, the base material for sheet forming is preferably composed of a thermoplastic resin having about 88.0 as the saponification value, for example, PVA and so forth.

The present invention is also a sheet type composition for diet comprising: the combination of the carbon-coated particles as described above, the essential oil adsorbing and desorbing agent, the released water absorption agent, the exothermic agent, the heat transfer inhibitor, the absorption promoter, the base for sheet forming, and sheet for contact-bonding.

Herein, the essential oil adsorbing and desorbing agent, the released water absorption agent, the exothermic agent, the heat transfer inhibitor, the absorption promoter, the base for sheet forming, and sheet for contact-bonding are the same as those explained above. In the sheet for thermal bonding, Kasenshi paper of which basis weight is about 18 to about 20 g is preferably used.

The present invention is a plaster type agent for diet comprising (1) a carbon-coated particle is produced by covering an essential oil adsorbent with neroli oil to prepare an essential oil-coated particle, and then further covering the particle by an essential oil adsorbing and desorbing regulator to prepare a carbon-coated particle (NR); and (2) a carbon-coated particle is produced by covering an essential oil adsorbent with jasmine oil to prepare an essential oil-coated particle, and then further covering the particle by an essential oil adsorbing and desorbing regulator to prepare a carbon-coated particle (JS); and (3) a carbon-coated particle is produced by covering an essential oil adsorbent with lavender oil, and then further covering the particle by an essential oil adsorbing and desorbing regulator to prepare a carbon-coated particle (LV).
In combination of these essential oil- coated particles, the safe and highly effective plaster agent for diet can be produced.

In the plaster agent for the diet of the present invention, addition to the above-mentioned essential oil-coated particles (1) to (3), the following two carbon-coated particles are preferably included: (4) the carbon-coated particle is produced by covering an essential oil adsorbent with rose oil to prepare an essential oil-coated particle, and then further covering the particle by an essential oil adsorbing and desorbing regulator to prepare a carbon-coated particle (RO), and (5) a carbon-coated particle is produced by covering an essential oil adsorbent with lemon oil to prepare an essential oil-coated particle, and then further covering the particle by an essential oil adsorbing and desorbing regulator to prepare a carbon-coated particle (LM).

Instead of the combination as described above, the plaster for the diet of the present invention may be comprises (1) a carbon-coated particle is produced by covering an essential oil adsorbent with neroli oil to prepare an essential oil-coated particle, and then further covering the particle by an essential oil adsorbing and desorbing regulator to prepare a carbon-coated particle (NR); and (2) a carbon-coated particle is produced by covering an essential oil adsorbent with jasmine oil to prepare an essential oil-coated particle, and then further covering the particle by an essential oil adsorbing and desorbing regulator to prepare a carbon-coated particle (JS); and (3) a carbon-coated particle is produced by covering an essential oil adsorbent with any one of essential oil selected form the group consisting of cineol type Eucalyptus oil, gai oil, and sage oil, to prepare an essential oil-coated particle (CE), (4) the carbon-coated particle is produced by covering an essential oil adsorbent with rose oil to prepare an essential oil-coated particle, and then further covering the particle by an essential oil adsorbing and desorbing regulator to prepare a carbon-coated particle (RO), and (5) a carbon-coated particle is produced by covering an essential oil adsorbent with lemon oil to prepare an essential oil-coated particle, and then further covering the particle by an essential oil adsorbing and desorbing regulator to prepare a carbon-coated particle (LM).

Furthermore, in addition to the combination of the above-mentioned essential oil-coated particles, the plaster for diet of the present invention preferably further comprises the absorption prompter and the base. It is preferable that the absorption promoter is L-menthol or limonene.

It is preferably that the base is an oleaginous base selected form the group consisting of fatty acid ester such as lard, beef tallow, fatty oil, paraffin having branched chain, solid paraffin, white soft paraffin, caproic acid, enanthic acid, caprylic acid, pelargonic acid, undecylic acid, lauric acid, tridecylic acid, myristic acid, pentadecylic acid, palmitic acid, heptadecylic acid, stearic acid, oleic acid, myristyl palmitate, stearyl stearate, myristic myristate, ceryl lignocerate: lanolin, lately describing waxes; glyceryl laurate, glyceryl monomyristate, glyceryl monooleate, glyceryl monostearate, glyceryl dilaurate, glyceryl dimyristate, glyceryl distearate, glyceryl trilaurate, glyceryl trimyristate, glyceryl tristearate; soybean oil, camellia oil, rape seed oil, peanut oil, sesame oil, safflower oil, mink oil, egg yolk oil, squarane, fish oil, whale oil, liver oil, carnauba wax, yellow beeswax , and white beewax.

Alternatively, it is preferably that the base is a water-soluble base selected from the group consisting of carboxyvinylpolymer, hydroxypropylcellulose, macrogol, and methylcellulose. When said carboxyvinylpolymer is used as said water-soluble base, diisopropylethanolamine and diisopropyladipate are used as neutralization agent.

In another aspect, the present invention is a method for producing a percutaneous type pharmaceutical agent for diet comprising steps of: forming essential oil-coating by weighing predetermined weight of essential oil according to claim 1, and mixing the oil with an essential oil adsorbent to allow a coating of the essential oil on the essential oil adsorbent; forming carbon-coated particle by adding a porous carbon material which adsorbs and desorbs an essential oil, to an essential oil-coated adsorbent to allow a carbon-coated particle being coated by the porous carbon material, after the coating of essential oil was formed; forming a composition for diet by mixing homogeneously a predetermined amount of the carbon-coated particle, and a base material for sheet forming, to prepare a layer having even thickness to form the composition for diet by heating; thermal-bonding by cutting said composition for diet agent, which is percutaneous agent, in a predetermined size of a piece to sandwich it by using two sheet type materials, and then thermal-bonding of four sides of the sheet type materials.

Furthermore, the present invention is a method for producing a plaster type pharmaceutical preparation for diet comprising the steps of: forming essential oil coating by weighing predetermined weight of said essential oil according to claim 12, and respectively mixing the essential oil with an essential oil adsorbent to allow a coating of the essential oil on the essential oil adsorbent; forming a carbon-coated particle by adding a porous carbon material, which adsorbs and desorbs an essential oil, to an essential oil-coated adsorbent to allow a carbon-coated particle being coated by the porous carbon material, after the coating of essential oil was formed; forming a mixture for a plaster agent by mixing homogeneously a predetermined amount of the carbon-coated particle, an absorption promoter, and either one of an oleaginous base or water-soluble base to form a mixture thereof, and forming a plaster by spreading the mixture on a sheet made of paper or plastic.

According to the above-mentioned producing method, each essential oil being adsorbed on the essential oil adsorbent to coat the surface of the adsorbent by using the method is covered by the carbon particles, and then the particles covered by the carbon are enveloped with sheet type materials. As a result, a percutaneous type pharmaceutical preparation can be produced, wherein the essential oils, active ingredients of the pharmaceutical preparation, do not directly contact with skin, but they are released in a sustained manner from the preparation during long time.
Furthermore, by using the method, a sheet type percutaneous pharmaceutical agent or plaster for diet including active ingredient in a desirable amount can be obtained.

### EFFECT OF THE INVENTION

As explained above, the percutaneous preparations of the present invention has no side effect that is shown in the administration of the conventional preparation for the diet. Also, the administration of the preparation of the present invention is easily discontinued by taking off it from the application site. Furthermore, length of treatment is not limited. According to the method of the present invention, percutaneous type pharmaceutical preparation for diet is conveniently produced. Particularly, the present invention has an effect for sustained release by using the carbon-coated particle that is covered by the carbon, which functions as adsorbing and desorbing agent for the essential oil.

According to the composition for the percutaneous type pharmaceutical agent of the present invention, the composition can be cut in a desired size, a pharmaceutical preparation including proper dose for a patient to be administered can be produced.
Further, according to the method for producing the composition for the percutaneous type preparation for diet can be produced by changing the contained amounts of the carbon-coated particles.

### Brief Description of Drawings

Fig. 1 shows the decrease of leptin amount in plasma when OB-A to OB-C, and OBT-A to OBT-C are applied;
Fig. 2 shows the decrease of abdominal panniculus adiposus weight when OB-A to OB-C, and OBT-A to OBT-C are applied;
Fig. 3 shows the gain of body weight for general feeding staff intake groups and high fat feeding staff intake groups;
Fig. 4 shows the difference of abdominal fat weight between the standard amount of OBT-A or OBT-B applied groups and their double amount applied groups; and
Fig. 5 shows the difference of leptin amount in plasma when either of the standard amount or double amount of OBT-A is applied, or those of OBT-B is applied.

### BEST MODE FOR CARRYING OUT THE INVENTION

The present invention is explained in detail herein below.
Neroli oil used in the present invention is obtained by steam distillation from flowers of *Citrus aurantium* L. subsp. *amara Engel* that is mainly produced in France, Italy, Spain, Morocco and Algeria. Yield of the neroli oil is about 0.1 %. As the components contained therein, 1-linalol and linalyl acetate (the sum of these two ingredients are about 35 to 40 %), α-terpineol, geraniol, geranyl acetate, neloridol (several %), and terpenes such as α-pinene, dipentene, camphene and ocimene, and sulfur-containing compounds such as methyl anthranilate and indole are mentioned. In the present invention, the oil obtained from *Citrus aurantium* L. subsp. *amara Engel* produced in France or Spain is preferably used, because they contain geraniol and nerolidol.

Jasmine oil used in the present invention is obtained from Jasminum officinale L. or J. officinale L. var. grandiflorum belonged to Oleaceae family that is mainly grown in south France, Italy, Egypt or Morocco.

When the flower is extracted with solvent, concrete is obtained; then the concrete is treated with alcohol to obtain jasmine absolute. As the components contained therein, benzyl acetate (65 %), d-linalol (15.5 %), linalyl acetate (7.5 %), jasmone (inherent component), methyl jasmonate (inherent component), jasmine lactone (inherent component), benzyl alcohol, methyl anthranilate, indol, nerol, geraniol and so forth are mentioned. In the present invention, jasmin produced in Morocco is preferably used, because it contains jasmine lactone.

Lavender oil is obtained by steam distillation from flowers of *Lavandula officinalis* Chaix. which is mainly produced in France, Italy, Hungary, southern part of ex USSR, England, north America, Australia, and Hokkaido of Japan. As the components contained therein, linalol (10 to 20 %), linalyl acetate (30 to 60 %), lavandulol, lavandulyl acetate, 3-octanol, α-pinene, β-pinene, limonene, cineol, and citroneral and other components are mentioned. In the present invention, Lavender produced in Italy, Hungary and France are preferably used, because it contains lavandulyl in relatively large amount.

Lavandin is obtained by steam distillation of the flower of Lavandula hybrida Reverch produced in south France. L. hybrida Reverch. is generated by crossing Lavender and Spike Lavender (L. latifolia Villars) . As the components contained therein, linalol, linalyl acetate, linalol oxide, cineol, d-campher, d-lavandulol and so forth are mentioned. In the present invention, lavandin produced in south France is preferably used, because it contains linalyl acetate and cineol in relatively large amount.

Rose oil is obtained from flower of damask rose, Rosa damascena Mill. forma trigintipetala Dieck produced in Bulgaria, Turk, Russia and, Rosa centifolia L. produced in France, Rosa damascena Mill. produced in Syria and Morocco.
At the last minutes of blooming, the rose is picked and immediately subject to steam distillation, thereby rose oil (Otto of rose, Attar of rose) is obtained in a yield from 0.01 to 0.04. Instead of steam distillation, when extraction with petroleum ether is performed, rose concrete is obtained in a yield from 0.22 to 0.25 %. From the concrete, Absolute is obtained in the yield from 50 to 60 %.

As the components contained therein, 1-citronellol (hereinbelow, "1" is sometimes described as "L") and phenyletyl alcohol, main components, and geraniol, nerol, linalool, farnesol, rose oxide and damascenone, these two components are important components, and damascone, ionone, stearoptene, methyl eugenol, and the like.

In the present invention, the rose absolute as described above is preferably used, because the quality of the essential oil and supply are stable. Furthermore, that produced in France is more preferably, because it has high phenyletyl alcohol content.

Cineol type *Eucalyptus* oil is obtained from leaves of *Eucalyptus globulus* Labill. by steam distillation, originally come from Tasmania, is mainly gown in north America, Mexico, Africa, and south Spain, in a yield from 0.75 to 1.25 %.
As the components contained therein, cineol (about 70 to 80 %), α-pinene, camphene, pinocarveol, pinocarvone, myrcenol, berebenone, carvone, eudesmol, and C₄ to C₆ aliphatic aldehyde, and the like are mentioned.

In the present invention, the cineol type *Eucalyptus* oil as describe above is preferably used, because it has high 1,8-cineol content; particularly, the cineol type *Eucalyptus* oil produced in Australia is stable in quality, as well as its quality is high and has good cost performance.
As the essential oil describe above, commercially available ones supplied from Ogawa & Co., Ltd and so forth may be used.

As a positive control in order to confirm the effect of the pharmaceutical agent for the diet of the present invention, grapefruit oil, lemon oil and sweet orange oil, which are known that they have diet effect and used, are solely used for preparing the pharmaceutical agent.

Grapefruit oil is obtained from pericarp of grapefruits, *Citrus paradisi Macfayden* that is mainly produced in California, Florida, Texas, Israel and Brazil. When pericarp of grapefruit is expressed, grapefruit oil is obtained, and when leaves or branches are subjected to steam distillation, grapefruit petitgrain oil is obtained.
As the components contained therein, d-limonene at the ratio more than 90 %, nootkatone as the inherent component, octylaldehyde, citral, geraniol and acetate ester thereof are mentioned. For a reference example, grapefruits oil that contains octyl aldehyde produced in California is preferably used.

Lemon oil is obtained by expression from pericarp of lemon, *Citrus limone* (L.), which is mainly produced in California, Sicily, Calabria, Spain and Brazil as well as is cultivated in Japan. It comprises, for example, d-limonene, citral, octyl aldehyde, nonyl aldehyde, linalol, geraniol, and other various terpenoid compounds. For a reference example, the essential oil from lemon that contains nonyl aldehyde produced in California is preferably used.

Sweet orange oil is obtained by expressing from fruits of *Citrus sinensis Osbeck* var. *brasiliensis* Tanaka as a whole, which is grown in worldwide, for example, California, Florida, Spain, Brazil, Italy and Japan, ant then separating the sweet orange oil and fruit juice. As the components contained therein, for example, limonene, citral, n-decylaldehyde, d-linalol, terpineol, and n-nonylalcohol are mentioned, and the content of d-limonene is more than 90 %. For a reference example, the essential oil from sweet orange produced in California is preferably used, because of the n-decyl aldehyde contents.

As the grapefruit oil, lemon oil, and sweet orange oil describe above, commercially available ones supplied from Ogawa & Co., Ltd and so forth may be used.

L-menthol (5-methyl 2-(1-methylethyl) cyclohexanol) is usually called menthol (Hakkanou). It has 12 isomeric forms in chemical, but only natural or synthetic L-menthol have cool aroma that is characteristic for menthol. L-menthol becomes a colorless column crystal or needle crystal soluble in ethanol, but insoluble in water. It gradually is sublimed at room temperature.

In order to obtain natural menthol, mentha oil is cooled, and precipitated crystalline is separated by centrifugation. Synthetic one is made of d-citronellal obtained from fractional distillation of citronella oil. d-Citronellal is converted into *l*- isopulegol, and hydrogenated to obtain the synthesized menthol. Alternatively, it is obtained by using either one method as described above: in one method, myrcene, which is obtained from pinene, is used as a raw material, to firstly obtain optically-active citronellal by using a specific catalyst, and menthol is asymmetrically synthesized, not performing an optical fractionation. On the other hand, menthol is also obtained by the optical fractionation of menthol mixture obtained from hydrogenated thymol.

Limonene (p-mentha-1,18-diene) is a monocyclic monoterpene hydrocarbon derived from menthane, and it has two enantiomers d-form and l-form, which is sometimes described as "L-form". Limonene is a liquid having lemon-like fragrance, and insoluble in water. In limonene, d-form is contained in orange peel oil, lemon oil, bergamot oil, fennel oil and the like; l-form is contained in pine needle oil, mint oil and the like. A racemic form of limonene is called as dipentene, and it has high content in turpentine oil or camphor oil.
d-Limonene is obtained by steam distillation of pericarp of orange, lemon and the like, and then the obtained oil is subject to fractional distillation. l-Limonene is obtained by fractional distillation of Japanese mint oil, which is obtained from whole plants of Japanese mint grown in Hokkaido, Okayama, Brazil, Paraguay, China and the like. Dipentene is obtained by fractional distillation of camphor oil.

The essential oil adsorbent used in the percutaneous type of the agent for diet of the present invention is defined as a resin to be a carrier for the essential oils as described above, and a polyvinyl alcohol (PVA) type water-absorptive resin having the saponification value in the range of 98.0 to 98.5 is preferably used. If the saponification value is less than 98.0, the surface of the carrier is gelatinized and loses functions as the adsorbent carrier. However, its saponification value is in the range of 98.0 to 98.5, the surface is not gelatinized and maintains the functions as the adsorbent carrier.
Specifically, there are mentioned, for example, Shin-Etsu Poval C-17GP, Poval A (produced by Shin-Etsu Chemical Co., Ltd.), and so forth, and Shin-Etsu Poval C-17GP or Poval A is preferably used.

The released water absorption agent used in the percutaneous type agent for diet of the present invention is defined as an absorption agent to remove the water existed on the skin on which the percutaneous type agent is applied. An acrylic type water-absorptive resin is preferably used, because such resin has high performance of water absorption in general, and also has good adhesive properties when it is subjected to a heating process for producing the composition described in below.

The acrylic type water-absorptive resin may absorb water generated on the particular space of the skin surface during the percutaneous agent is applied on, and not limited to a particular resin. It is preferably used the resin being capable of absorbing 400 to 800 times of its volume of water based on the volume of the water-absorptive resin. If the capacity of the water-absorption is lower that 400 times, the resin is not capable of absorbing entire of the generated water, but the capacity over 800 times is not necessary. For example, there are mentioned Sanfresh (Sanyo Chemical Industries. Ltd.), Aquakeep (Registered trademark, Sumitomo Seika Chemicals Co., Ltd.), and the like. Among them, Sanfresh having fractured form is preferably used because it has good adhesive property compared with Aquakeep having particle form.

An essential oil adsorbing and desorbing regulator used in the percutaneous pharmaceutical agent for diet of the present invention is defined as a porous carbon material that coats the surface of the layer formed by the above-mentioned essential oil on the essential oil adsorbent for regulating adsorption and desorption of the essential oil. Specifically, there are mentioned, for example, activated charcoal that adsorbs a variety of molecules. The activated charcoal having 200 to 1,000 m²/g of surface area is preferably used; because the amount of the essential oil adsorbed to the charcoal per weight is a little and thereby desorbing the essential oil becomes easy. The activated charcoal having 400 to 800 m²/g of surface area may be more preferably used.
In the present invention, as the above-mentioned activated charcoal, which is finely divided particle, commercially available one may be used. There are mentioned, for example, Shirasagi P (Takeda Pharmaceutical Company Ltd.) and so forth. Among them, Shirasagi P is preferably used, because the adsorption area is not too large and the cost is reasonable.

The base material for sheet forming used in the percutaneous type agent for diet the present invention is defined as the base material to form a sheet type diet composition. It contains an exothermic agent, a heat transfer inhibitor, an absorption promoter and a thermoplastic resin.

The exothermic agent used in the percutaneous agent for diet of the present invention is defined as a material that adsorbs moisture in the air and then generates adsorption heat. By using the thermal energy generated when the moisture is adsorbed, the essential oil adsorbed onto the carrier adsorbent is desorbed. Specifically, there is mentioned zeolite as an example. Among the zeolite, synthetic one without metal and has pore size from 0.1 to 0.8 nm is preferably used, because the energy for adsorption and desorption is properly supplied. The zeolite has pore size from 0.3 to 0.4 nm is more preferably used. Commercially available zeolite may be used when it has such pore sizes, and specifically, Zeolum (Tosoh Corporation) and so forth are mentioned as examples.

The heat transfer inhibitor is defined as a compound for inhibiting to transfer the heat, which is caused by the free-water adsorption onto the free-water adsorbent. Specifically, there are mentioned, for example, polysaccharide compound such as Chitosan and cellulose and so forth.
When a dye is contained in the pharmaceutical preparation, use of Chitosan gives an advantage that Chitosan may be used as the carrier of such a dye. In order to inhibit the heat transfer, cellulose and the like are used instead of Chitosan.

The absorption promoter is defined as monoterpene that functions to improve to absorb the essential oil in the agent. As the absorption promoter, L-menthol and other terpene compound are specifically mentioned as the example, and commercially available one may be used. Among them, L-menthol has the advantageous effect that it removes the free-water remained on the skin, and then arranges circumstances for the essential oil to be absorbed through the skin.

The thermoplastic resin having the saponification value, about 88.0, is preferably used. In order to form the percutaneous agent for diet, the composition for the agent should be subjected to a heating process. In this time, the resin should have preferable properties that neither release the essential oils from the carbon coated particle nor stuck spaces among the resin particle, even though the adhesion is performed under lower temperature, such as about 180°C.
Specifically, for example, Gohselan L-0301 (Registered trademark, Nippon Synthetic Chemical Industry Co., Ltd.) is preferably used, because of the good adhesive properties at low temperature about 180°C.

The percutaneous agent for diet of the present invention may be prepared as plaster form agent after mixing the essential oil-coated particles, and then the particles are mixed with hydrophobic oleaginous base used for preparing ointment or suppository, or hydrophilic base easily soluble in water among the base defined in the Japanese Pharmacopoeia

As the oleaginous base, there are mentioned, for example, lard, beef tallow, fatty oil, hydrocarbons, higher alcohols, higher fatty acid, higher fatty acid esters, glycols, plant oils, animal oils and so forth.
Among them, as the hydrocarbon, there are mentioned, for example, liquid paraffin that is a mixture of a variety of the hydrocarbons, paraffin having branched chain (Commercial name, Isopar, registered trademark, Exxon Mobil Corporation), solid paraffin, white soft paraffin, and so forth.

As the higher fatty acid, there are mentioned, for example, caproic acid, enanthic acid, caprylic acid, pelargonic acid, undecylic acid, lauric acid, tridecylic acid, myristic acid, pentadecylic acid, palmitic acid, heptadecylic acid, stearic acid, oleic acid, and so forth.
As the higher fatty acid ester, there are mentioned, for example, fatty acid ester such as myristyl myristate, myristyl palmitate, stearyl stearate, ceryl lignocerate: lanolin; lately describing waxes; glyceryl laurate, glyceryl monomyristate, glyceryl monooleate, glyceryl monostearate, glyceryl dilaurate, glyceryl dimyristate, glyceryl distearate, glyceryl trilaurate, glyceryl trimyristate, glyceryl tristearate, and so forth.

As the fatty oil, there are mentioned, for example, the plant oil such as soybean oil, camellia oil, rape seed oil, peanut oil, sesame oil, and safflower oil; animal oil such as mink oil, egg yolk oil, squarane, fish oil, whale oil, and liver oil; and hardened oil produced by hydrogenation thereof is also mentioned.

As the wax, there are mentioned, for example, carnauba wax, yellow bee wax, white beeswax, and so forth. As the petrolatum, there are mentioned, for example, yellow petrolatum, white soft paraffin and so forth.
White soft paraffin is preferably used, because it is compatible to almost pharmaceutical drug with no change. According to the description on the Japan Pharmacopoeia, white soft paraffin is combined with white bee wax and sorbitan sesquioleate to prepare white ointment and then absorption promoter is properly added to prepare the plaster.

Alternatively, as the water soluble base, there are mentioned, for example, carboxyvinylpolymer, hydroxypropylcellulose (herein below, it is also referred to as simply "HPC"), macrogol, methylcellulose, and so forth.
A commercially available water-soluble base, Hiviswako (Registered trademark, Wako Pure Chemical Industries, Ltd.) is preferably used. When Polyethylene glycol 400 and Polyethylene glycol 4000 are mixed at equal ratio, the macrogol ointment is prepared and may be used as the water-soluble base. Alternatively, HPC having good compatibility with propylene glycol may be used as the base.
Note that skin irritation is reduced by adding, at least, diisopropylethanolamine and diisopropyladipate as neutralization agents, when an acidic water-soluble polymer is used as the base.

Since the main ingredient of the present invention is aliphatic essential oils obtained from plants, for example, L-menthol or limonene may be preferably used, are combined with oleaginous base, if necessary. When the main ingredient is combined with the water-soluble base, the absorption promoter is not necessary. However, menthol may be used in the amount as mentioned above.

The percutaneous agent for diet of the present invention is prepared by using the essential oil, the essential oil adsorbent, the free-water remover, the essential oil adsorbing and desorbing regulator, the exothermic agent, the heat transfer inhibitor, the absorption promoter, and the base material for sheet forming, as described above. The method for producing thereof is explained in below.

At first, a desired amount of the essential oil is weighed, and then it is mixed with PVA to coat the surface of PVA particle in a proper size vessel. Subsequently, the desired amount of the activated charcoal is weighed and added to cover the surface of the essential oil-coated PVA to prepare the charcoal-coated particle.

Next, Zeolum which is the exothermic agent, chitosan which is the heat transfer inhibitor, L-menthol which is the absorption promoter, Sanfresh which is the base material for sheet forming, and activated charcoal are homogenously mixed in the desirable ratio to prepare a base for the agent. Wherein, in order to improve formability of the sheet type composition lately described, the additional activated charcoal is added separately from those used for forming the carbon-coated particles.
After that, the mixture is spread out on a sheet for contact bonding and then another sheet for contact bonding covers them to be heated to prepare the sheet type of the composition for the percutaneous agent for the diet of the present invention.

After the mixture is sandwiched by the sheets for contact bonding, the sandwiched mixture is preferably heated from about 160 °C to about 200 °C, more preferably about 180 °C. By heating the sandwiched mixture at the temperature of the above-mentioned range, the base material for sheet forming, the resin, is adhered each other, keeping spaces among them. Since the spaces works as isles for the molecule of the essential oil from the charcoal coated particle to the skin surface, on which the percutaneous anxiolytic of the present invention is applied, the molecule of the essential oil is delivered to the skin and absorbed percutaneously. On the other hand, when the mixture is heated about 230 °C or higher temperature than it, the spaces among the resin are filled, because the resins are melted. Therefore, the molecule of the essential oil described above is incapable of delivering to the skin.

As the sheet for contact bonding, Kasenshi paper (basis weight 18 to 20 g) is preferably used, and the use of Kasenshi paper has the advantage that the ratio of contact bonding of carbon coated particles is high.
The sheets contact bonded to the composition is sandwiched with two sheet type materials made of nonwoven fabrics with proper sizes, for example, 2 x 4 cm; and then four sides of the sheets are heat-sealed to prepare a piece of the percutaneous agent for diet of the present invention.

Alternatively, the sheet type material is preferably selected from the group consisting of paper, woven fabric and nonwoven fabric. Among them, the nonwoven fabric is more preferable because of its good permeability.
The essential oil contained in the percutaneous agent is released as gaseous state, and it flows in the spaces among the particles of the base material for sheet forming to go out by permeating the fabric.

Plaster agents are prepared in below. At first, the essential oils are weighed at the desirable amounts, and mixed with PVA to coat the surface of PVA. After that, PVA with the essential oil coated is mixed with the desirable amount of the activated charcoal as described above to form the carbon-coated particle A.
Subsequently, the desirable amount of menthol is weighed, and it is refereed to as the particle B.

These particles A and B are mixed, and after the addition of menthol, the mixture is further mixed with the oleaginous base or the water-soluble base, and spread on a sheet made of the paper, the plastic film, and so forth.
As explained above, the composition for the diet and the agent for diet of the present invention are prepared.
Alternatively, as the reference examples, three agents including sole essential oil instead of the above-mentioned combination of the three carbon-coated particles but others are prepared as the same as described above. In this way, the reference agents may be prepared.

Note that the sheet type percutaneous type agent and the plaster are mentioned in the above-described embodiment. However, the agent is prepared as an ointment by using generally used base for preparing the ointment with the essential oil coated particles and absorption promoter, or cream and so forth.

### EXAMPLES

The present invention will now be described by the following examples in detail. However, the present invention is not limited to the

### examples.

### (EXAMPLE 1)

### (1) Reagents

In order to produce the composition for the percutaneous type agent for diet and the percutaneous agent (the examples), positive control agent (the reference examples), and placebo (the comparative examples), the following reagents were used.

### (1-1) Essential oils

Neroli oil, jasmine oil, lavender oil, rose oil, cineol type Eucalyptus oil, and L-menthol is purchased from Ogawa & Co., Ltd. Grapefruits oil, lemon oil and sweet orange oil are also purchased from Ogawa & Co., Ltd.

### (1-2) Others

As PVA, Shin-Etsu Poval C-17GP was purchased from Shin-Etsu Chemical Co., Ltd., and Goselan L-3031 was purchased from Nippon Synthetic Chemical Industry Co., Ltd. As acrylic type of water-absorptive resin, Sanfresh (registered trademark) was purchased from Sanyo Chemical Industries, Ltd. As activated charcoal, Shirasagi P was purchased from Takeda Pharmaceutical Company Limited. As Zeolite, Zeolum (Registered trademark) is purchased from Tosoh Corporation. As Chitosan, Koyo Chitosan was purchased from Koyo Chemical Company Limited. Kasenshi paper is purchased from Nippon Daishowa Paperboard Co,. Ltd., and the nonwoven fabric is purchased from Nisshinbo Industries, Inc.

### (EXAMPLE 2) Preparation of the composition for diet and percutaneous type agent for diet

### (2-1) Preparation of the carbon-coated particle

The carbon coated particle was prepared according to the recipe shown in the following table 1. Note that each piece of the agent was regulated so as to contain 1.1 mg of each carbon-coated particle prepared by using the amount shown in the table 1, when the pharmaceutical agents (Name of the agent is OBT-A, OBT-B, and OBT-C) were prepared.

**(Table 3)**

| Contents(weight(mg)) | | Combination of carbon coated particle | | |
|---|---|---|---|---|
| | | A | B | C |
| Kinds of carbon coated particles | neroli oil | 17 | 11 | 11 |
| | jasmine oil | 13 | 11 | 11 |
| | lavender oil | 70 | 37 | - |
| | rose oil | - | 15 | 15 |
| | cineol type Eucalyptus oil | - | - | 37 |
| | lemon oil | - | 26 | 26 |
| P V A(Shin-Etsu Poval) | | 120 | 120 | 120 |
| Activated Charcoal | | 3 | 3 | 3 |
| Total | | 223 | 223 | 223 |

For example, in the combination A, neroli oil (N), jasmine oil (J) and lavender oil (L) were separately weighed at the amount shown in the table 3, and put them separately in three different 500 ml of a transparent closed vessel made of glass. PVA (Shin-Etsu Poval C-17GP) was added into the vessel and mixed with each essential oil at the room temperature to prepare the essential oil-coated particles. The carbon coated particles prepared as mentioned above were then stored in different tight sealed vessels separately, for example, a glass vessel with a ground-glass stopper, at room temperature.

Then, each of the carbon-coated particles was separately weighed in the amount mentioned in the table 3 to prepare the combination of the particles A, B and C. These A, B, and C were corresponding to the name of the agent OBT-A, OBT-B, and OBT-C.
Alternatively, the base described in the following table 4 was prepared in parallel with the preparation of the combination of the carbon-coated particles A, B, and C as described above.

**(Table 4)**

| Contents | | Ratio (%) |
|---|---|---|
| Base | Sanfresh | 58 |
| | L-menthol | 27 |
| | Zeolum | 9 |
| | Koyo Chitosan | 5 |
| | Activated Charcoal | 1 |
| | Total | 100 |

In order to prepare the base, at first, Sanfresh (Sanyo Chemical Industries, Ltd.), Zeolum (zeolite, Tohsoh Corporation), L-menthol (Ogawa & Co., Ltd.), and Koyo Chitosan (Koyo Company Ltd.) were sequentially in this order added and mixed, and prepared.

### (2-2) Preparation of the composition for the percutaneous agent for the diet

### (1) Preparation of the composition for the percutaneous agent for the diet (single dose (standard), hereinafter, it is referred to as "x 1")

Three kinds of carbon-coated particles prepared in the above (2-1) were mixed with the base and other components of the amounts shown in Table 4 to prepare the percutaneous type agent for diet (x 1).
Namely, the combination of the essential oil-coated particles A, B, and C shown in Table 3, the base shown in Table 5, and activated charcoal for forming, chitosan for forming, and PVA were mixed in the amounts shown in Table 5, and prepared each of the mixture, OBT-A, OBT-B, or OBT-C of the agent of the present invention.

Subsequently, the composition was spread on the sheet for contact bonding so as to have even thickness, and then another sheet for contact bonding was placed on them to sandwich the mixture. The sandwiched mixture is then subjected to contact bonding under heating, about 180°C, and formed as the sheet type composition for diet agent. The sheet type composition was cut in a size of about 2 x 4 cm, or about 1 x 2 cm to form pieces and sandwiched with the nonwoven fabric that is also cut in the proper size to cover the piece. After that, four sides of the nonwoven fabric was heat sealed to prepare the percutaneous agent for the diet.

The recipe including the combination of the carbon coated particle A shown in Table 3 was named as OBT-A, including that of B was named as OBT-B, and including that of C was named as OBT-C, respectively.
Note that the loss rate of the essential oil during the contact bonding described later was calculated as 15 %.

**(Table 5)**

| Name of Agent | Recipe (mg) | | |
|---|---|---|---|
| | OBT-A | OBT-B | OBT-C |
| Carbon coated particle | 100 | 100 | 100 |
| Activated charcoal for forming | 3 | 3 | 3 |
| Chitosan for forming | 7 | 7 | 7 |
| PVA | 120 | 120 | 120 |
| Base | 80 | 80 | 80 |
| Total | 310 | 310 | 310 |

### (2) Preparation of the percutaneous type agent for the diet (double amount, it is referred to as " x 2"

Except including the combination of the carbon coated particle in the amounts shown in Tables 6 and 7, the percutaneous type agents for the diet including the double amount of the active ingredient were prepared similarly to the above (1).

**(Table 6)**

| Amounts (weight(mg)) | | Combination of carbon coated particles | | |
|---|---|---|---|---|
| | | A2 | B2 | C2 |
| kinds of carbon coated particles | neroli oil | 34 | 22 | 22 |
| | jasmine oil | 26 | 22 | 22 |
| | lavender oil | 140 | 74 | - |
| | rose oil | - | 30 | 30 |
| | cineol type Eucalyptus oil | - | - | 74 |
| | lemon oil | - | 52 | 52 |
| PVA (Shin-etsu poval) | | 120 | 120 | 120 |
| Activated charcoal | | 3 | 3 | 3 |
| Total | | 323 | 323 | 323 |

**(Table 7)**

| Name of Agents | Recipe (mg) | | |
|---|---|---|---|
| | OBT-A2 | OBT-B2 | OBT-C2 |
| Carbon coated particles | 200 | 200 | 200 |
| Activated charcoal for forming | 3 | 3 | 3 |
| Chitosan for forming | 7 | 7 | 7 |
| PVA | 120 | 120 | 120 |
| Base | 80 | 80 | 80 |
| Total | 410 | 410 | 410 |

### (2-3) Preparation for the positive control agent (reference example agent)

### (1) Preparation of the reference agent (x 1)

By using grapefruits oil, lemon oil, or sweet orange oil, according to the protocol as described above, the carbon-coated particles corresponding to the essential oils used were prepared. The agent, which solely includes one of the carbon-coated particle was used instead of the combination of the carbon-coated particles, was prepared as reference examples. The agent including the carbon coated particles of grapefruits was names as OB-A, that of lemon oil was named as OB-B, and that of sweet orange oil was named as OB-C. The carbon coated particles included in three reference example agents and their composition were shown in the following Table 8 and 9.

**(Table 8)**

| Amounts (weight(mg)) | | Carbon-coated particles | | |
|---|---|---|---|---|
| | | A | B | C |
| kinds of carbon coated particles | grapefruits oil | 100 | - | - |
| | lemon oil | - | 100 | - |
| | sweet orange oil | - | - | 100 |
| PVA (Shin-etsu poval) | | 120 | 120 | 120 |
| Activated charcoal | | 3 | 3 | 3 |
| Total | | 223 | 223 | 223 |

**(Table 9)**

| Name of agent | Amounts (g) | | |
|---|---|---|---|
| | OB-A | OB-B | OB-C |
| Essential oil | 100 | 100 | 100 |
| Activated charcoal | 3 | 3 | 3 |
| Chitosan | 7 | 7 | 7 |
| PVA | 120 | 120 | 120 |
| Base | 80 | 80 | 80 |
| Total | 310 | 310 | 310 |

### (2) Preparation for the reference example (x 2)

Except including the combination of the carbon coated particle in the amounts shown in Tables 10 and 11, the percutaneous type agents for the diet including the double amount of the active ingredient (x 2) were prepared similarly to the above (1).

**(Table 10)**

| Amounts (weight (g)) | | Carbon-coated Particles | | |
|---|---|---|---|---|
| | | A2 | B2 | C2 |
| Kinds of carbon coated particles | grapefruits oil | 200 | - | - |
| | lemon oil | - | 200 | - |
| | sweet orange oil | - | - | 200 |
| PVA (Shin-etsu poval) | | 120 | 120 | 120 |
| Activated charcoal | | 3 | 3 | 3 |
| Total | | 323 | 323 | 323 |

**(Table 11)**

| Name of Agent | Amounts (g) | | |
|---|---|---|---|
| | OB-A2 | OB-B2 | OB-C2 |
| Carbon coated particle | 200 | 200 | 200 |
| Activated charcoal for forming | 3 | 3 | 3 |
| Chitosan for forming | 7 | 7 | 7 |
| PVA | 120 | 120 | 120 |
| Base | 80 | 80 | 80 |
| Total | 410 | 410 | 410 |

### (2-4) Preparation of the negative control agent (Placebo or comparative example agent)

Placebo was prepared as the same as mentioned above, using black paper not including essential oil coating particles instead of the sheet type composition of the present invention.

### (EXAMPLE 3) Evaluation of the diet effect by each percutaneous agent including essential oils

Evaluation of the diet by the percutaneous agents including each essential oils was performed by using two indexes: weight change of the panniculus adiposus of the abodomen and level change of leptin in plasma.

### (3-1) Test animals and so forth

As the test animals, 5 weeks age of ICR mice were purchased from Tokyo Laboratory animals Co. Ltd. These mice were kept under the conditions 10 mice per cage, at 24 ± 1 °C, 12 hours light-dark cycle (turn on the lights at 8 o'clock and turn off the lights at 20:00 o'clock) for 1 week, taking fed and water freely.
After that, the agents for the diet of the present invention (Examples), the reference agents (Reference example), and placebo agents were assigned to different cages, and these agents were adhered for 14 days repeatedly.
Pentobarbital was purchased from Dainippon Pharmaceutical Co., Ltd. In order to determine the leptin level in the plasma, Leptin/Mouse ELISA Kit was purchased from Morinaga Institute of Biological Science Inc.

### (3-2) Test Method

On the beginning day of adhesion of the agents, the mice were anesthetized by using pentobarbital (70mg/kg, i. p.), and then shaved hair around waistline. On the same day, the agent for diet, the reference agent, or the placebo agent was adhered on the every mice of each group as assigned under right ether anesthesia. These agents adhered were replaced to new agents every day, for 14 days. After 24 hour from the final adhesion, the mice were decapitated to collect blood and the panniculus adiposus of the abodomen.

The blood was collected from the cervical part decapitated into Eppendorf tube (1.5 ml of volume size) in which its inner surface was wetted by heparin solution. The tube was immediately turned upside down to prepare blood samples. Then, the blood samples were centrifuged for 15 minutes at 10,000 x g at 4°C, and the separated plasma were stored in a frozen state in a freezer at -80°C.
By using the Mouse/leptin ELISA kit, the leptin levels in the plasma samples were determined.
The panniculus adiposus of the abodomen was collected by removing the adiposus around inguinal area of both legs. Two adipose tissues obtained form each mouse were weighed together.

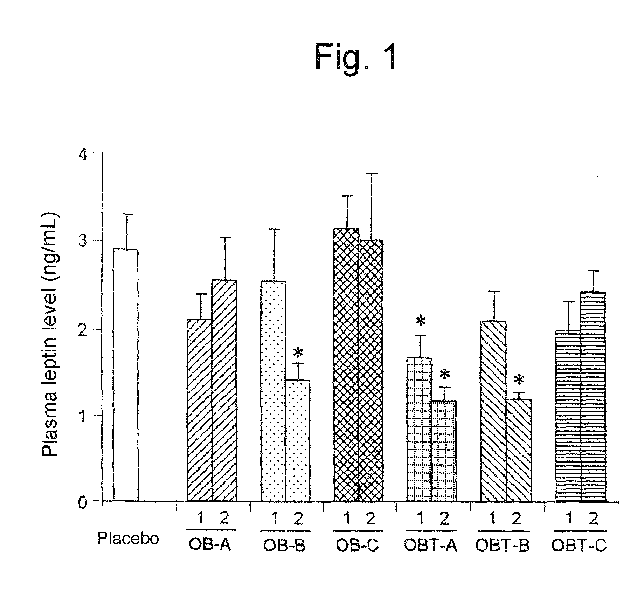

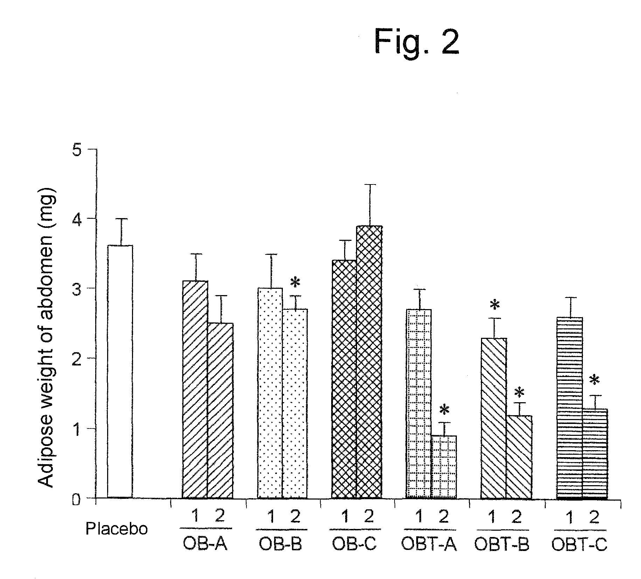

Obtained data were subjected to comparison between groups: the placebo agent adhesion group and the reference example agent adhesion group, the placebo agent adhesion group and the diet agent adhesion group.
In order to confirm dispersion, the data was analyzed by F-test. If the significant difference was not less than 5 %, the data was analyzed by using Student t-test for further evaluation; if it is less than 5 %, the data was analyzed by using Aspin-Welch t-test for further evaluation. When p < 0.05 in these two t-test, it was decided that there was significant difference between two groups compared, and showed by asterisk in Figs 1 and 2.

The effect to the leptin level in the plasma was shown in Fig. 1. In Fig. 1, "1" shows the standard of each agent and "2" shows these including double amounts of the standard.
As shown in Fig. 1, the significant decrease of the leptin level in plasma was observed only when the double amounts of OB-B (x 2) was adhered. On the other hand, the standard of OBT-A (x 1), the double amount of it (x 2), and the double amounts of OBT-B (x 2) showed the significant decrease. It was demonstrated that these agents have effect to decrease plasma leptin level.

Effect for the panniculus adiposus tissues of the abdomen was shown in Fig. 2. In Fig. 2, "1" shows the standard of each agent and "2" shows these including double amounts of the standard.
As shown in Fig. 2, the significant decrease was observed only when the double amounts of OB-B (x 2) was adhered similarly to the plasma leptin level. On the other hand, no significant decrease of the weight of the panniculus adiposus by the standard of OBT-A was observed, although it showed the significant decrease of the plasma leptin level. However, the double amounts of OBT-A significantly decreased the panniculus adiposus. In OBT-B, the significant decrease of the weight of the panniculus adiposus of the abdomen by either of the standard or the double amounts. Furthermore, even in OBT-C, which did not show the significant decrease of the plasma leptin level, the weight of the panniculus adiposus of the abdomen was significantly decreased when the double amounts of OBT-C was adhered.

Accordingly, it was demonstrated that OBT-A to OBT-C had the advantageous effect to decrease the plasma leptin level or the weight of panniculus adiposus of the abdomen, comparing to OBT-A to OBT-C, which include one essential oil.

### (EXAMPLE 4) Diet effect for obesity mice caused by eating high-fat feeding staff

### (4-1) Test animal and so forth

As the test animals, 4 weeks age of ICR mice were purchased from Tokyo Laboratory animals Co. Ltd. These mice were kept under the conditions 10 mice per cage, at 24 ± 1 °C, 12 hours light-dark cycle (turn on the lights at 8 o'clock and turn off the lights at 20:00 o'clock) for 1 week, taking fed and water freely.

As a high-fat feeding staff, High Fat Diet 32 was purchased from Crea Japan Inc, and as a normal feeding staff, MF was purchased from Oriental Yeast Co., Ltd. Pentobarbital was purchased from Dainippon Pharmaceutical Co., Ltd. In order to determine the leptin level in the plasma, Leptin/Mouse ELISA Kit was purchased from Morinaga Institute of Biological Science Inc.
As the agent for diet of the present invention, as mentioned above, the standard of OBT-A (OBT-A x 1) and the double amounts of it (OBT-A x 2), the standard of OBT-B (OBT-B x 1) and the double amounts of it (OBT-B x 2) were used. As the placebo agent, the control agent shown in the (EXAMPLE 2-4) was used.

### (4-2) Test method

As a test group, ICR mice described above was used after being fed the high-fat feeding staff for 4 weeks and induced the adipositas.
The mice were fed either of the above-mentioned feeding staff for 4 weeks, and then they received each of the agents for the diet by adhesion. The groups were named the test group A x1, A x 2, B x 1, and B x 2. The negative control group was gave the normal feeding staff and the control agent by adhesion, and the positive control group gave the high-fat feeding staff and the control agent by adhesion.

All of the mice of the groups were weighed everyday. On day 28 from beginning of feeding either of the feeding staff to the mice, the mice were anesthetized by using pentobarbital (70mg/kg, i. p.), and then shaved their hair around waistline. The agents were adhered on shaved site of the mice under right ether anesthesia. These agents adhered were replaced to new agents every day, for 14 days.

After 24 hour from the final adhesion of the agent, the mice were decapitated to collect blood and the panniculus adiposus of the abodomen.
The blood was immediately turned upside down in the tube, and centrifuged for 15 minutes at 10,000 x g at 4°C to separate, and the separated plasma were stored in the frozen state in a freezer at -80°C as the sample.

The plasma leptin level was determined by using the Mouse/leptin ELISA kit similarly to the EXAMPLE 3. The panniculus adiposus tissues were weighed.

### (4-3) Results

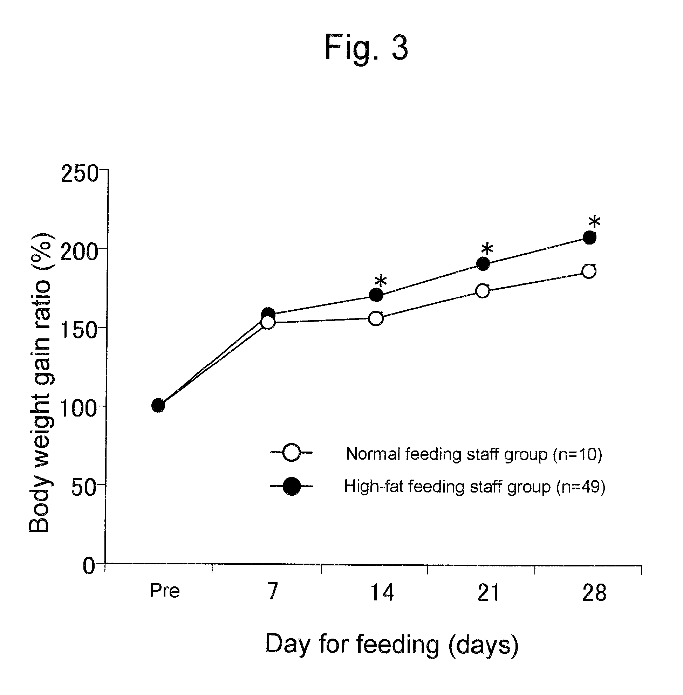

As shown in Fig. 3, after 14 day from the beginning of feeding, the significant difference was observed between the normal feeding staff group and the high-fat feeding staff group.

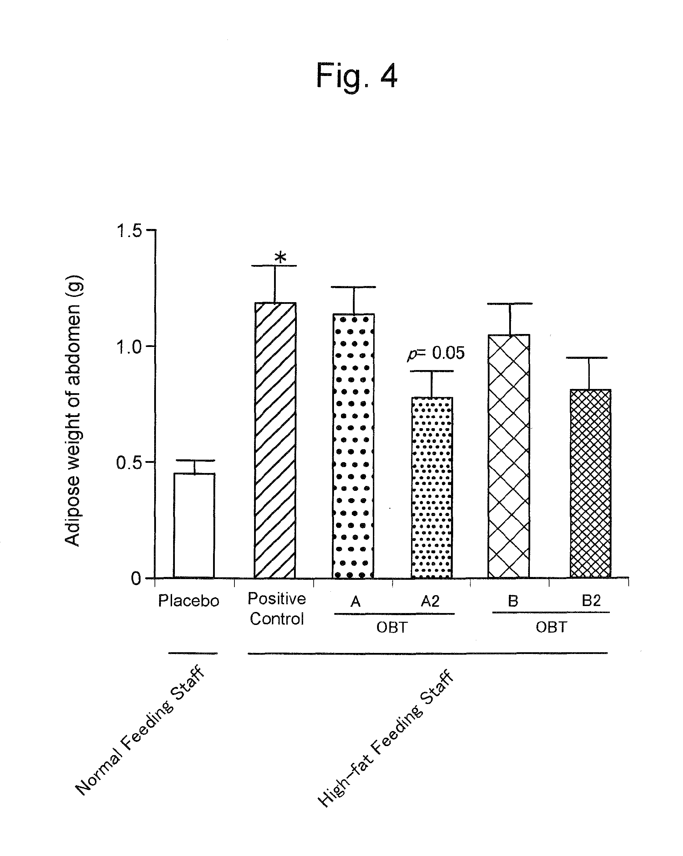

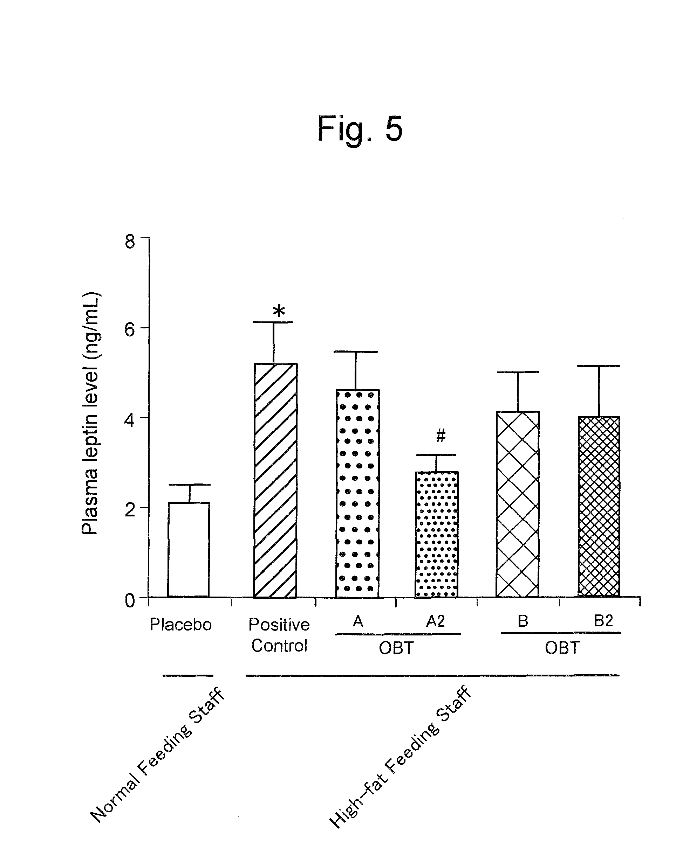

As shown in Fig. 4, the adipose weight of the abdomen was significantly different between the negative control group and the positive control group. All of the mice in the agents for the diet adhered group showed lower weight compared to that of the mice in the positive control group. In Fig. 4, the group shows significant difference was gave asterisk (p<0.05).
As shown in Fig. 5, the same tendency was observed in the plasma leptin level. In B x 1 group and B x 2 group, there were no significant difference compared to the positive control group, and between B x 1 and B x 2 groups.
Between the positive control group and A x 1 group, there was no significant difference. However, between the positive control group and A x 2 group, there was significant difference, and a x 2 suppressed the increase of the plasma leptin level significantly (p < 0.05). In Fig. 5, the group shows significant difference was gave asterisk or #.

As a result, it was demonstrated that the double amounts of OBT-A showed the decrease of plasma leptin level or weight of panniculus adiposus of the abdomen, even in the adipositas was induced by feeding the high-fat feeding staff.

### INDUSTRIAL APPLICABILITY

According to the agent for diet of the present invention, it is available in the medical field related to obesity prevention or treatment by decreasing the plasma leptin level or loosing weight of panniculus adiposus.

## Claims

1. A composition for diet comprising at least following essential oil-coated particles, comprising:
(1) a carbon-coated particle which is produced by covering an essential oil adsorbent with neroli oil to prepare an essential oil-coated particle, and then further covering the particle by an essential oil adsorbing and desorbing regulator to prepare a carbon-coated particle; and
(2) a carbon-coated particle which is produced by covering an essential oil adsorbent with jasmine oil to prepare an essential oil-coated particle, and then further covering the particle by an essential oil adsorbing and desorbing regulator to prepare a carbon-coated particle.

2. The composition for diet according to claim 1, further comprising a carbon-coated particle which is produced by covering an essential oil adsorbent with lavender oil to prepare an essential oil-coated particle, and then further covering the particle by an essential oil adsorbing and desorbing regulator to prepare a carbon-coated particle.

3. The composition for diet according to claim 1 or 2, further comprising a carbon-coated particle which is produced by covering an essential oil adsorbent with rose oil to prepare an essential oil-coated particle, and then further covering the particle by an essential oil adsorbing and desorbing regulator to prepare a carbon-coated particle.

4. The composition for diet according to claim 3, further comprising a carbon-coated particle which is produced by covering an essential oil adsorbent with lemon oil to prepare an essential oil-coated particle, and then further covering the particle by an essential oil adsorbing and desorbing regulator to prepare a carbon-coated particle.

5. The composition for diet according to claim 3, further comprising
a carbon-coated particle which is produced by covering an essential oil adsorbent with any one of essential oil selected from the group consisting of cineol type Eucalyptus oil, gai oil, and sage oil, to prepare an essential oil-coated particle, and then further covering the particle by an essential oil adsorbing and desorbing regulator to prepare a carbon-coated particle; and
another carbon-coated particle which is produced by covering an essential oil adsorbent with rose oil to prepare an essential oil-coated particle, and then further covering the particle by an essential oil adsorbing and desorbing regulator to prepare a carbon-coated particle.

6. The composition for diet according to claim 1, wherein
an amount of neroli oil used to prepare said carbon-coated particle which is in the range from 9 to 21 weight % against a total amount of essential oils used to prepare the composition; and
the amount of jasmine oil used to prepare said carbon-coated particle which is in the range from 9 to 15 weight % against a total amount of essential oils used to prepare the composition.

7. The composition for diet according to claim 2, wherein
the amount of lavender oil used to prepare said carbon-coated particle is in the range from 65 to 75 weight % against a total amount of essential oils used to prepare the composition;
the amount of neroli oil used to prepare said carbon-coated particle is in the range from 15.5 to 20.5 weight % against a total amount of essential oils used to prepare the composition; and
the amount of jasmine oil used to prepare said carbon-coated particle is in the range from 9.5 to 14.5 weight % against a total amount of essential oils used to prepare the composition.

8. The composition for diet according to claim 5, wherein
the amount of lavender oil used to prepare said carbon-coated particle or that of any one of essential oil selected from the group consisting of cineol type Eucalyptus oil, gai oil, and sage oil, is in the range from 32 to 42 weight % against a total amount of essential oils used to prepare the composition;
the amount of neroli oil used to prepare said carbon-coated particle is in the range from 10 to 12 weight % against a total amount of essential oils used to prepare the composition;
the amount of jasmine oil used to prepare said carbon-coated particle is in the range from 10 to 12 weight % against a total amount of essential oils used to prepare the composition;
the amount of lemon oil used to prepare said carbon-coated particle is in the range from 24.5 to 27 weight % against a total amount of essential oils used to prepare the composition; and
the amount of rose oil used to prepare said carbon-coated particle is in the range from 13.5 to 17 weight % against a total amount of essential oils used to prepare the composition.

9. The sheet type composition for diet according to claim 1, wherein said essential oil adsorbent is a polyvinyl alcohol type water absorption resin of which saponification value is from 98.0 to 98.5.

10. The sheet type composition for diet according to claim 1, further comprising:
said an essential oil adsorbing and desorbing regulator is a porous carbon material having surface area from 200 to 800 m²/g;
said released water absorption agent is an acrylic type water-absorptive resin being capable of absorbing 400 to 800 times to its volume of water based on the volume of the water-absorptive acrylic resin;
an exothermic agent composed of a zeolite having a pore size from 0.1 to 0.8 nm;
said heat transfer inhibitor comprising a polysaccharide compound;
said absorption promoter comprising L-menthol or limonene; and
said base material for sheet forming comprising a thermoplastic resin having about 88.0 as the saponification value.

11. A sheet type composition for diet comprising:
against the total weight of the composition,
the carbon-coated particle according to claims 5 to 7, in a range from 28.0 to 52.8 weight %;
said essential oil adsorbing and desorbing agent in the range from 2.08 to 3.63 weight %;
said released water absorption agent in the range from 27.5 to 41.0 weight %; and,
said base material for sheet forming in the range from 17.6 to 27.0 weight %.

12. A sheet type medical agent for diet comprising: said essential oil coated particle according to claim 1, said essential oil adsorbing and desorbing regulator, said released water absorption agent, said base material for sheet forming, and a sheet for contact bonding.

13. A plaster type agent for diet comprising
(1) a carbon-coated particle which is produced by covering an essential oil adsorbent with neroli oil to prepare an essential oil-coated particle, and then further covering the particle by an essential oil adsorbing and desorbing regulator to prepare a carbon-coated particle; and
(2) a carbon-coated particle which is produced by covering an essential oil adsorbent with jasmine oil to prepare an essential oil-coated particle, and then further covering the particle by an essential oil adsorbing and desorbing regulator to prepare a carbon-coated particle; and
(3) a carbon-coated particle which is produced by covering an essential oil adsorbent with any one of essential oil selected form the group consisting of cineol type Eucalyptus oil, gai oil, and sage oil, to prepare an essential oil-coated particle, and then further covering the particle by an essential oil adsorbing and desorbing regulator to prepare a carbon-coated particle.

14. The plaster type agent for diet according to claim 12, further comprising
a carbon-coated particle which is produced by covering rose oil to prepare an essential oil-coated particle, and then further covering the particle by an essential oil adsorbing and desorbing regulator to prepare a carbon-coated particle.

15. The plaster type agent for diet comprising
(1) a carbon-coated particle which is produced by covering an essential oil adsorbent with neroli oil to prepare an essential oil-coated particle, and then further covering the particle by an essential oil adsorbing and desorbing regulator to prepare a carbon-coated particle;
(2) a carbon-coated particle which is produced by covering an essential oil adsorbent with jasmine oil to prepare an essential oil-coated particle, and then further covering the particle by an essential oil adsorbing and desorbing regulator to prepare a carbon-coated particle;
(3) a carbon-coated particle which is produced by covering an essential oil adsorbent with one of essential oil selected form the group consisting of cineol type Eucalyptus oil, gai oil, and sage oil, to prepare an essential oil-coated particle, and then further covering the particle by an essential oil adsorbing and desorbing regulator to prepare a carbon-coated particle; and
(4) a carbon-coated particle which is produced by covering an essential oil adsorbent with rose oil to prepare an essential oil-coated particle, and then further covering the particle by an essential oil adsorbing and desorbing regulator to prepare a carbon-coated particle.

16. The plaster type agent for diet according to claim 12, further comprising
said absorption promoter including L-menthol or limonene; and
either of any one oleaginous base selected from the group consisting of fatty acid ester such as lard, beef tallow, fatty oil, paraffin having branched chain, solid paraffin, white soft paraffin, caproic acid, enanthic acid, caprylic acid, pelargonic acid, undecylic acid, lauric acid, tridecylic acid, myristic acid, pentadecylic acid, palmitic acid, heptadecylic acid, stearic acid, oleic acid, myristyl palmitate, stearyl stearate, myristic myristate, ceryl lignocerate: lanolin, lately describing waxes; glyceryl laurate, glyceryl monomyristate, glyceryl monooleate, glyceryl monostearate, glyceryl dilaurate, glyceryl dimyristate, glyceryl distearate, glyceryl trilaurate, glyceryl trimyristate, glyceryl tristearate; soybean oil, camellia oil, rape seed oil, peanut oil, sesame oil, safflower oil, mink oil, egg yolk oil, squarane, fish oil, whale oil, liver oil, carnauba wax, yellow beeswax , and white beewax; or
any one of water-soluble base selected from the group consisting of carboxyvinylpolymer, hydroxypropylcellulose, macrogol, and methylcellulose.

17. A plaster type agent fro diet according to claim 15, wherein diisopropylethanolamine and diisopropyladipate are used as neutralization agents, when said carboxyvinylpolymer is used as said water-soluble base.

18. A method for producing a percutaneous type pharmaceutical agent for diet comprising steps of:
forming essential oil-coating by weighing predetermined weight of essential oil according to claim 1, and mixing the oil with an essential oil adsorbent to allow a coating of the essential oil on the essential oil adsorbent;
forming carbon-coated particle by adding a porous carbon material which adsorbs and desorbs an essential oil, to an essential oil-coated adsorbent to allow a carbon-coated particle being coated by the porous carbon material, after the coating of essential oil was formed;
forming a composition for diet by mixing homogeneously a predetermined amount of the carbon-coated particle, and a base material for sheet forming, to prepare a layer having even thickness to form the composition for diet by heating;
thermal-bonding by cutting said composition for diet agent, which is percutaneous agent, in a predetermined size of a piece to sandwich it by using two sheet type materials, and then thermal-bonding of four sides of the sheet type materials.

19. A method for producing a plaster type pharmaceutical preparation for diet comprising the steps of:
forming essential oil coating by weighing predetermined weight of said essential oil according to claim 12, and respectively mixing the essential oil with an essential oil adsorbent to allow a coating of the essential oil on the essential oil adsorbent;
forming a carbon-coated particle by adding a porous carbon material, which adsorbs and desorbs an essential oil, to an essential oil-coated adsorbent to allow a carbon-coated particle being coated by the porous carbon material, after the coating of essential oil was formed;
forming a mixture for a plaster agent by mixing homogeneously a predetermined amount of the carbon-coated particle, an absorption promoter, and either one of an oleaginous base or water-soluble base to form a mixture thereof, and
forming a plaster by spreading the mixture on a sheet made of paper or plastic.
